# EUROPÄISCHE PATENTANMELDUNG

(11) **EP 0 813 856 A2**
(43) Veröffentlichungstag der Anmeldung: **29.12.1997**
(21) Anmeldenummer: 97250185.2
(22) Anmeldetag: 16.06.1997
(51) Int. Cl.: A61K 6/083

(54) **Dentalmaterialien auf der Basis von polymerisierbaren Wachsen**

(30) Priorität: 18.06.1996 DE 19626356
(71) Anmelder: IVOCLAR AG, 9494 Schaan (LI)
(72) Erfinder: Völkel, Thomas, Dr., 88131 Lindau (DE); Zanghellini, Gerhard, 9494 Schaan (LI); Fischer, Urs Karl, 9320 Arbon (CH); Moszner, Norbert, Prof. Dr., 9492 Eschen (LI); Rheinberger, Volker, Dr., 9490 Vaduz (LI)
(74) Vertreter: UEXKÜLL & STOLBERG

(57) **Zusammenfassung**

Die Erfindung betrifft Dentalmaterialien auf der Basis von wachsartigen polymerisierbaren Substanzen. Die Dentalmaterialien enthalten (a) 0 bis 70 Gew.-% mindestens eines polymerisierbaren Monomers und/oder Oligomers; (b) 0,1 bis 5 Gew.-% mindestens eines Polymerisationsinitiators; (c) 0 bis 60 Gew.-% eines oder mehrerer Füllstoffe, (d) mindestens 20 Gew.-% der wachsartigen polymerisierbaren Substanz. Sie sind bei Raumtemperatur knetbar und lassen sich im nicht-polymerisierten Zustand ohne zusätzliches Erwärmen wie herkömmliche Wachse modellieren. Bei der Polymerisation wird die Wachskomponenten kovalent in das Polymer eingebunden. Die Dentalmaterialien eignen sich zur Herstellung temporärer und permanenter Dentalprothesen.

## Beschreibung

Die vorliegende Erfindung betrifft Dentalmaterialien auf der Basis von polymerisierbaren Wachsen, die sich besonders als Materialien für temporäre und permanente Dentalprothesen, Inlays und Kronen eignen.

Der Begriff Wachs stellt eine Sammelbezeichnung für eine Reihe von natürlichen und künstlichen Stoffen dar. Im allgemeinen werden hierunter Materialien verstanden, die schon unterhalb ihres Schmelz- oder Erweichungsbereichs leicht plastisch verformbar sind, ein durchscheinendes bis opakes Aussehen aufweisen, oberhalb von 40 °C ohne Zersetzung schmelzen und schon wenig oberhalb des Schmelzpunktes verhältnismäßig niedrigviskose Flüssigkeiten ergeben und die eine stark temperaturabhängige Konsistenz und Löslichkeit besitzen. Darüber hinaus können Wachse meist unter leichtem Druck poliert werden (Ullmanns Enzyklopädie der Technischen Chemie, 4. Auflage, Band 24, Verlag Chemie, Weinheim 1989).

Im Dentalbereich finden Wachse als Modellier-, Guß-, Biß- oder Klebewachse Anwendung. Hierbei kommen fast ausschließlich Mischungen aus Naturwachsen und synthetischen Wachsen zum Einsatz, wobei sich die physikalischen Eigenschaften der Wachse nach der gewünschten Verwendung richten (K. Körber, K. Ludwig, Zahnärztliche Werkstoffe und Technologie, G. Thieme Verlag, Stuttgart 1982, Seite 90). Beispielsweise sollte ein zum Modellieren von Formteilen geeignetes Modellierwachs eine leichte Verformbarkeit und bei Mundtemperatur eine möglichst gute Formbeständigkeit aufweisen. Hierzu eignen sich Mischungen aus Paraffin-, Stearin-, Japan- und Bienenwachs.

Konventionelle Wachse werden zur Verarbeitung im allgemeinen erwärmt. Das Aushärten erfolgt durch das Erstarren des Wachses beim anschließenden Abkühlen. Hierbei treten in der Regel relativ große, unkontrollierbare Volumenänderungen auf. Darüber hinaus weisen konventionelle Wachse den Nachteil auf, daß sie beim Entfernen vom Modell leicht brechen und sich schon unter relativ geringer mechanischer und vor allem thermischer Belastung verformen.

Zur Vermeidung dieser Nachteile werden neuerdings Mischungen aus Natur- und teil- bzw. vollsynthetischen Wachsen mit polymerisierbaren Monomeren oder Oligomeren wie polyfunktionellen Acrylaten und Methacrylaten eingesetzt. Diese Mischungen lassen sich nach der Bearbeitung gezielt härten und weisen anschließend eine höhere mechanische Stabilität als die reinen Wachse auf.

Beispielsweise werden in der JP-A-92/4748 (C.A. 120 (1993) 136156) und der JP-A-91/330047 (C.A. 119 (1993) 282306) Mischungen aus Polyethylenwachsen mit Styrol bzw. Acrylatmonomeren offenbart. Weiterhin werden in der JP-A-90/312256 (C.A. 128 (1992) 27523) photopolymerisierbare Wachse für den Dentalbereich auf der Basis von Mischungen aus Natur- bzw. synthetischen Wachsen mit herkömmlichen Acrylverbindungen und Photoinitiatoren beschrieben. Nachteilig an diesen Mischungen ist, daß herkömmliche Dentalwachse und dentale Vernetzermonomere sich schlecht miteinander vermischen und daß vor allem das Wachs bei der Polymerisation nicht in das Polymernetzwerk eingebunden wird. Es kann daher zur Ausbildung von Wachs-Domänen kommen, d.h. es bildet sich ein mehrphasiges System, dessen mechanische Festigkeit dementsprechend gering ist.

Die EP-B-0 110 193 offenbart wachsfreie Wachsersatzstoffe zur Herstellung von Gußmodellen. Diese Zusammensetzungen basieren auf Mischungen aus di- oder polyfunktionellen Meth- oder Acrylsäureestern, einem Photoinitiator und gegebenenfalls einem Photoaktivator sowie organischen Füllstoffen. Aufgrund der Zusammensetzung der in der EP-B-0 110 193 beanspruchten Materialien zeigen diese keine wachsartigen Eigenschaften, d.h. daß die Materialien nicht wie Wachse modellierbar sind und dementsprechend der Zahntechniker die übliche Wachstechnik durch eine aufwendige Schicht-Technik ersetzen muß. Dabei können Änderungen bzw. Korrekturen am Modell nicht mehr vorgenommen werden, da nach dem Auftragen der jeweiligen Schicht ihre Härtung durch Polymerisation erfolgt. Darüber hinaus ist bei diesem Verfahren von Nachteil, daß dünne Schichten nur mit wenig- oder ungefüllten Mischungen aus di- oder polyfunktionellen (Meth)acrylsäureestern hergestellt werden können, bei deren Polymerisation es zu einer erheblichen Volumenkontraktion kommt.

Die EP-B-0 380 116 offenbart Mischungen, die zur Beeinflussung des Expansionsverhaltens der Formmassen zusätzlich eine organische Verbindung mit einem Siedepunkt und/oder einem Sublimationspunkt im Bereich oberhalb von 150 °C enthalten, welche keine Reaktion mit der organischen polymerisierbaren Masse eingehen. Die genannten Zusammensetzungen zeichnen sich insbesondere dadurch aus, daß sie rückstandsfrei verbrennbar sind.

Die US-A-5 403 188 offenbart thermoplastische Formmassen, die sich zur Herstellung dentaler Abdrücke eignen. Die Massen enthalten ein polymeres thermoplastisches Material, vorzugsweise ein Polycaprolacton, ein polymerisierbares Harz, einen Initiator und ggf. einen Füllstoff. Sie sind bei 38°C fest und praktisch nicht verformbar und werden zur Verarbeitung erwärmt. Die Aushärtung erfolgt anschließend durch Abkühlen. Nach dem Herstellen des Abdrucks werden die Massen zusätzlich durch eine radikalische Polymerisation gehärtet. Die Materialien weisen den Nachteil auf, daß sie nur zu hochviskosen Produkten aufschmelzen. Eine zahntechnische Formgebung ist ausgesprochen schwierig, da die Materialien im geschmolzenen Zustand nicht zur zäh-viskos sondern auch sehr klebrig sind.

Aufgabe der Erfindung ist die Schaffung von wachsartigen Dentalmaterialien, welche die oben genannten Nachteile nicht aufweisen und sich insbesondere auch zur Herstellung temporärer und permanenter Prothesen eignen.

Die Aufgabe wird durch Dentalmaterialien auf der Basis von polymerisierbaren Wachsen gelöst, welche
a) 0 bis 70 Gew.-%, vorzugsweise 5 bis 70 Gew.-%, besonders bevorzugt 5 bis 60 Gew.-% und ganz besonders bevorzugt 20 bis 50 Gew.-% mindestens eines polymerisierbaren Monomers und/oder Oligomers;
b) 0,1 bis 5 Gew.-%, vorzugsweise 0,2 bis 2,0 Gew.-% mindestens eines Polymerisationsinitiators;
c) 0 bis 60 Gew.-%, vorzugsweise 0 bis 50 Gew.-% und besonders bevorzugt 0 bis 30 Gew.-% Füllstoffe; und
d) mindestens 20 Gew.-%, vorzugsweise mindestens 40 Gew.-% einer wachsartigen polymerisierbaren Substanz
enthalten.

Geeignete polymerisierbare Monomere und Oligomere sind beispielsweise aus der DE-C-3 941 629 oder DE-A-4 029 230 bekannt. Bevorzugt sind vernetzend wirkende Verbindungen wie Di-, Tri- oder Tetraethylenglycoldi(meth)acrylat, Decandioldi(meth)acrylat, Bisphenol-A-di(meth)acrylat, Trimethylolpropantri(meth)acrylat, Pentaerythrittetra(meth)acrylat, 2,2-Bis(4-methacryloyloxy-2-hydroxypropoxy)-phenylpropan (Bis-GMA) und das Umsetzungsprodukt von 1 Mol 2,2,4-Trimethylhexamethylendiisocyanat mit 2 Mol 2-Hydroxyethyl(meth)acrylat sowie Mischungen dieser Monomere.

Geeignete Polymerisationsinitiatoren sind beispielsweise aus der DE-A 4 029 230 bekannt. Bevorzugte Initiatoren sind Campferchinon, 9,10-Phenanthrenchinon oder Diacetyl. Die Polymerisationsinitiatoren können darüber hinaus mit einem geeigneten Reduktionsmittel kombiniert werden. Hierzu eignen sich besonders 4-Dimethylaminobenzoesäureester, N,N'-(2-Cyanoethyl)methylanilin, Triethanolamin oder 2-(Dimethylamino)ethylmethacrylat.

Die erfindungsgemäßen Dentalmaterialien können bis zu 60 Gew.-% Füllstoffe enthalten, wobei jedoch Dentalmaterialien mit geringem oder ohne Füllstoffanteil bevorzugt sind. Die Füllstoffe dienen zur Erhöhung der mechanischen Festigkeit, zur Reduzierung des Polymerisationsschrumpfes und zur Kontrolle der Viskosität. Bevorzugte Füllstoffe sind beispielsweise aus der DE-C-3 941 629 bekannt. Besonders bevorzugt sind Füllstoffe in Form von amorphen kugelförmigen Materialien auf der Basis von Mischoxiden aus SiO₂, ZrO₂ und/oder TiO₂, mikrofeine Füllstoffe, wie pyrogene Kieselsäure oder Fällungskieselsäure sowie Makro- oder Minifüllstoffe, wie Quarz-, Glaskeramik- oder Glaspulver.

Als wachsartige polymerisierbare Substanzen sind solche Stoffe bevorzugt, die eine längerkettige Carbonsäure, ein Carbonsäurederivat, eine OH-funktionalisierte Verbindung und/oder ein Derivat einer OH-funktionalisierten Verbindung darstellen und die eine oder mehrere polymerisationsfähige Gruppen aufweisen. Bevorzugte Carbonsäurederivate sind die Ester mit polymerisationsfähigen Alkoholen, bevorzugte OH-funktionalisierte Verbindungen sind Alkohole. Als Derivate der OH-funktionalisierten Verbindungen sind die Ester mit polymerisationsfähigen Carbonsäurederivaten bevorzugt. Die wachsartigen polymerisierbaren Substanzen werden vorzugsweise in monomerer Form eingesetzt.

Geeignete wachsartige polymerisierbare Substanzen sind zum Teil kommerziell erhältlich, wie zum Beispiel Stearylacrylat (Schmelzpunkt 23 °C, Firma Christ Chem.), PEG-1000-Dimethacrylat (Schmelzpunkt 20 °C) und PEG-4000-Diacrylat (Schmelzpunkt 52 °C, beide Firma Polysciences, Inc.), oder lassen sich durch chemische Modifikation geeigneter Carbonsäuren und Alkohole herstellen.

Hierzu sind Carbonsäuren mit einem Schmelzpunkt von über 60°C, insbesondere solche mit einer Kettenlänge von 16 bis 32 Kohlenstoffatomen bevorzugt. Ganz besonders bevorzugt sind Palmitinsäure (Schmelzpunkt: 64°C), Stearinsäure (Schmelzpunkt: 69°C), Eicosansäure (C₂₀H₄₀O₂; Schmelzpunkt: 74-76°C), Docosansäure (C₂₂H₄₄O₂; Schmelzpunkt: 80-82°C), Tricosansäure (C₂₃H₄₆O₂; Schmelzpunkt: 75-83°C), Hexacosansäure (C₂₆H₅₂O₂; Schmelzpunkt: 87-89°C), Heptacosansäure (C₂₇H₅₄O₂; Schmelzpunkt: 88-89°C) oder Octacosansäure (C₂₈H₅₆O₂; Schmelzpunkt: 61-63°C). Mischungen dieser Carbonsäuren sind ebenfalls geeignet. Bevorzugt sind Mischungen aus Palmitin- oder Stearinsäure mit Hexacosan- oder Heptacosansäure. Ein besonders geeignetes Ausgangsmaterial zur Herstellung der erfindungsgemäßen wachsartigen polymerisierbaren Substanzen ist das Hoechst-Wachs S (Firma Hoechst, Schmelzpunkt 80°C), das eine Mischung aus längerkettigen aliphatischen Carbonsäuren (C₁₆ bis C₃₆) darstellt.

Als Alkohole sind längerkettige Alkohole mit einem Schmelzpunkt von über 55°C als Augangsmaterialien zur Herstellung der polymerisationsfähigen wachsartigen Substanzen bevorzugt, insbesondere solche mit einer Kettenlänge von 18 bis 32 Kohlenstoffatomen. Ganz besonders bevorzugt sind 1,2-Octadecandiol (C₁₈H₃₈O₂; Schmelzpunkt: 74-76°C), 1-Eicosanol ((C₂₀H₄₁OH; Schmelzpunkt: 64-66°C), 1-Docosanol (C₂₂H₄₅OH; Schmelzpunkt: 65-72°C) oder 1-Hexacosanol (C₂₆H₅₃OH; Schmelzpunkt: 79-81°C), sowie Hoechst-Wachs KST (Firma Hoechst, Schmelzpunkt 57-59°C; basierend auf OH-terminiertem Polyethylenoxid).

Ausgehend von den voranstehenden wachsartigen Carbonsäuren erfolgt die Einführung von polymerisationsfähigen Gruppen vorzugsweise durch Umsetzung mit geeigneten ungesättigten Verbindungen, insbesondere Vinyl-, (Meth)acryl- oder Allylverbindungen, nach den bekannten Methoden der organischen Chemie. Polymerisationsfähige Methacrylatgruppen lassen sich beispielsweise nach Aktivierung der COOH-Gruppen z.B. mit Chlorameisensäureester nach der Methode der gemischten Anhydride in einer Eintopfreaktion durch Umsetzung mit 2-Hydroxyethylmethacrylat (HEMA), Hydroxypropylmethacrylat oder Glycerindimethacrylat (GDMA) einführen. Darüber hinaus kann die Einführung von Methacrylgruppen auch durch direkte Umsetzung der wachsartigen Carbonsäuren mit Methacrylsäure-2,3-epoxypropylester (GMA) erfolgen.

Ausgehend von den voranstehenden wachsartigen OH-funktionalisierten Verbindungen ist die Einführung von polymerisationsfähigen Gruppen wie z.B. Methacrylgruppen beispielsweise durch ihre einfache azeotrope Veresterung mit Methacrylsäure oder durch Acylierung mit Methacrylsäurechlorid bzw. Methacrylsäureanhydrid möglich. Weiterhin ist die Umsetzung der OH-funktionalisierten Verbindungen mit 2-Isocyanatoethylmethacrylat (IEMA) besonders geeignet.

Analog dazu können die COOH- bzw. OH-funktionalisierten wachsartigen Ausgangsverbindungen auch mit anderen geeigneten ungesättigten Verbindungen umgesetzt werden, so daß anstelle von Methacryl-Resten andere polymerisationsfähige Gruppen, wie Acryl-, Allyl-, Vinyl-, Vinylether- oder Styryl-Gruppen eingeführt werden. Geeignete Reagentien sind z.B. 2-Hydroxyethylacrylat, Acrylsäure, Acrylsäurechlorid, Allylalkohol, 3-Buten-1-ol, 2-Hydroxyethylvinylether, 4-Hydroxymethylstyrol oder 4-Vinylbenzoesäure. Als polymerisationsfähige Gruppen bevorzugt sind Methacrylat-, Acrylat- und Styrylgruppen.

Zur Herstellung der erfindungsgemäßen Dentalmaterialien werden die Ausgangsstoffe in den angegebenen Mengen gemischt. Neben den genannten Komponenten können die Dentalmaterialien weitere Stoffe wie zum Beispiel Pigmente oder Farbstoffe enthalten. Darüber hinaus können Mischungen aus verschiedenen wachsartigen polymerisierbaren Substanzen eingesetzt werden.

Die erfindungsgemäßen Dentalmaterialien weisen eine wachsartige Konsistenz auf, d.h. sie sind bei Raum- bzw. Körpertemperatur knetbar bis duktil hart und lassen sich im nicht-polymerisierten Zustand ohne zusätzliches Erwärmen wie herkömmliche Wachse modellieren, plastisch verformen oder andersartig zerstörungsfrei bearbeiten. Sie zeigen ein opakes bis teilweise durchscheinendes Aussehen und sind unter leichtem Druck polierbar. Oberhalb von ca. 40°C ergeben sie gering viskose, tropfbare Schmelzen, die nicht zur Fadenbildung neigen.

Nach dem Modellieren können die Materialien durch Polymerisation gehärtet werden. Vorzugsweise erfolgt die Härtung durch Photopolymerisation. Die zur Auslösung der Photopolymerisation notwendige Wellenlänge des Lichtes hängt von dem verwendeten Photoinitiators ab, wobei Photoinitiatoren mit einer Anregungswellenlänge im Wellenlängenbereich von 390 bis 500 nm bevorzugt sind. Die Photopolymerisation kann stufenweise bei steigenden Temperaturen erfolgen, d.h. zunächst wird das Dentalmaterial bei Raumtemperatur durch Bestrahlen gehärtet und anschließend im Temperaturbereich von ca. 40 bis 80°C nachpolymerisiert, wodurch die Festigkeit der Materialien weiter zunimmt.

Ein besonderer Vorteil, der erfindungsgemäßen wachsartigen polymerisierbaren Substanzen liegt darin, daß sie sich durch einen geringen Polymerisationsschrumpf auszeichnen, was für die Dimensionsstabilität des Gesamtmaterials sehr vorteilhaft ist. Die reinen polymerisationsfähigen Wachse weisen vorzugsweise einen Polymerisationsschrumpf von maximal 2,2 Vol.-% auf, während z.B. Methylmethacrylat, das die Monomerkomponente der meisten Prothesenmaterialien darstellt, einen Volumenschrumpf von 20,7 Vol.-% aufweist.

Die Wachskomponente wird während der Polymerisation kovalent in das gebildete Polymer eingebunden, so daß ein einphasiges System entsteht. Die ausgehärteten Dentalmaterialien weisen daher nach der Polymerisation eine wesentlich größere Festigkeit als bekannte wachshaltige Materialien auf. Durch Kombination der erfindungsgemäßen wachsartigen polymerisierbaren Substanzen mit anderen, vorzugsweise vernetzend wirkenden Monomeren können Dentalmaterialien hergestellt werden, die nach der Polymerisation den E-Modul konventioneller Polymethylmethacrylat(PMMA-)-Materialien nahe kommen. So weist ein bevorzugtes Dentalmaterial aus 66,3 Gew.-% Wachsmonomer, 32,9 Gew.-% Pentaerythrittetraacetat und 0,8 Gew.-% Photoinitiatorkombination nach der Polymerisation einen E-Modul von ca. 1,9 GPa auf.

Die erfindungsgemäßen Dentalmaterialien zeigen eine wesentlich höhere Mundbeständigkeit als herkömmliche Materialien und eignen sich daher im Gegensatz zu bekannten Materialien auch zur Herstellung von temporären und permanenten Prothesen.

Die erfindungsgemäßen Dentalmaterialien erlauben eine deutliche Vereinfachung des Herstellungsprozesses z.B. von Totalprothesen. Bisher ist es bei der Herstellung von Totalprothesen üblich, zunächst eine Wachsprothese herzustellen, welche dem Patienten im Mund angepaßt wird. Sobald das Ergebnis zufriedenstellend ist, wird die Wachsprothese über die "Lost Wax Technik" in eine permanente PMMA-Prothese überführt. Dieser zusätzliche Verfahrensschritt kann bei Verwendung der erfindungsgemäßen Dentalmaterialien umgangen werden, da die Prothese direkt nach der Anpassung polymerisiert und ausgearbeitet werden kann. Dies bedeutet eine wesentliche Reduktion des Arbeitsaufwands und damit der bei der Herstellung von Prothesen anfallenden Kosten.

Ein weiteres Anwendungsgebiet der erfindungsgemäßen Dentalmaterialien liegt in der Herstellung temporärer Kronen, Brücken und Inlays. Die erfindungsgemäßen Dentalmaterialien können aufgrund ihrer guten Verformbarkeit im Mund des Patienten an den bearbeiteten Zahnstumpf angepaßt werden, wobei das Material durch einfaches Abschaben geformt und anschließend durch Polymerisation ausgehärtet werden kann. Zur Verstärkung insbesondere von Brücken können vor der Polymerisation Armierungen in das Material eingesetzt werden.

### Beispiele

### Beispiel 1: Synthese von Polymerisationsfähigen Wachsen durch Veresterung des Hoechst-Wachses S mit HEMA

Eine Mischung von 70 g Hoechst-Wachs S (186,7 mmol), 22,6 g Collidin (186,7 mmol), 20,3 g Chlorameisensäureethylester (186,7 mmol) und 1000 ml Toluol wird 72 h unter Feuchtigkeitsausschluß bei 60°C gerührt. Man gibt 24,4 g HEMA (186,7 mmol) und etwas p-Toluolsulfonsäure als Katalysator zu und rührt anschließend 3 Tage bei 60°C. Danach filtriert man den Reaktionsansatz bei 60°C und läßt das Filtrat 12 h im Kühlschrank bei 4°C stehen, wodurch ein weißer voluminöser Niederschlag gebildet wird, der nach dem Abtrennen und Waschen mit etwas angesäuertem Wasser und mit Ethanol im Exsikkator über wasserfreiem CaCl₂ getrocknet wird. Es werden ca. 60 g (Ausbeute 70%) eines wachsartigen Feststoffs erhalten (Schmelzbereich: 47 - 61°C, bestimmt durch Differential-Scanning-Calorimetrie (DSC)).

Die Viskosität des Wachses beträgt oberhalb des Schmelzbereichs ca. 0,5 Pa·s (gemessen bei 85°C; ab ca. 61°C nimmt die Viskosität nicht mehr signifikant mit der Temperatur ab).

Die Auswertung des ¹H-NMR-Spektrums zeigt, daß 48 % der COOH-Gruppen mit HEMA verestert sind.
¹H-NMR (CDCl₃): δ = 5,6 und 6,2 (2s, =CH₂), 4,3 (t, CH₂O), 2,2 (t, CH₂COO), 1,9 (s, CH₃) und 1,2 bis 1,4 ppm (breit, CH₂).

### Beispiel 2: Synthese eines polymerisationsfähigen Wachses durch Veresterung des Hoechst-Wachses S mit GDMA

Analog zu Beispiel 1 wird eine Mischung von 140 g Hoechst-Wachs S (373,4 mmol), 45,2 g Collidin (373,4 mmol), 40,5 g Chlorameisensäureethylester (373,4 mmol) und 1000 ml Toluol mit 85,2 g (373,4 mmol) GDMA umgesetzt. Nach der Aufarbeitung wurden 80 g (Ausbeute 50 %) eines wachsartigen Feststoffs erhalten (Schmelzpunkt 55 - 70°C).
¹H-NMR (CDCl₃): δ = 5,6 und 6,2 (2s, =CH₂), 4,0 bis 4,5 (m, CH₂O und CHO), 2,2 (t, CH₂COO), 1,9 (s, CH₃) und 1,2 bis 1,6 ppm (breit, CH₂).
IR (Film): 3450 (O-H), 2918 (C-H), 1737 (C=O), 1638 (C=C) und 1171 cm⁻¹(C-O).

### Beispiel 3: Synthese eines polymerisationsfähigen Wachses durch Umsetzung des Hoechst-Wachses S mit GMA

Eine Mischung von 40 g (0,11 mol) Hoechst-Wachs S, 44,6 g (0,31 mol) GMA, 0,7 g 1,4-Diazabicyclo[2.2.2]octan und 600 ml Toluol wird 5 Tage bei 65°C gerührt. Nach dem Einengen des Ansatzes auf ca. 200 ml läßt man die Mischung über Nacht im Kühlschrank stehen. Der gebildete Niederschlag wird abfiltriert, mit etwas angesäuertem Wasser und mit Ethanol gewaschen und anschließend im Exsikkator über wasserfreiem CaCl₂ getrocknet. Es ergeben sich ca. 51 g (Ausbeute: nahezu 100%) eines wachsartigen Feststoffs (Schmelzpunkt: 40-67°C durch DSC). Die Auswertung des ¹H-NMR-Spektrums zeigt, daß sich 90 % der COOH-Gruppen mit GMA umgesetzt haben.
¹H-NMR (CDCl₃): δ = 5,8 und 6,3 (2s, =CH₂), 4,3 bis 4,7 (m, HOCH-CH₂), 2,4 bis 2,6 (m, CH₂-C=O), 2,2 (s, CH₃) und 1,9 bis 2,1 ppm (breit, CH₃).
IR (Film): 3368 (O-H), 2927 (C-H), 1719 (C=O) und 1638 cm⁻¹ (C=C).

### Beispiel 4: Synthese eines polymerisationsfähigen Wachses durch Umsetzung von Octadecandiol-1,2 mit IEMA

7,8 g (50,3 mmol) IEMA werden zu einer Lösung von 7,4 g (25,6 mmol) Octadecandiol-1,2 und 20 mg Metatin 812 in 80 ml THF so zugetropft, daß die Temperatur nicht 26°C übersteigt. Nach fünftägigem Rühren wird das Lösungsmittel im Vakuum abdestilliert, wobei 14,6 g (Ausbeute: 97%) eines wachsartigen Feststoffs erhalten werden (Schmelzpunkt: 42°C).

| | | | | |
|---|---|---|---|---|
| C₃₂H₅₆N₂O₈ (596,8) | Ber.: | C 64,42 | H 9,53 | N 4,65 |
| | Gef.: | C 64,38 | H 9,41 | N 4,61 |

¹H-NMR (CDCl₃): δ = 5,6 und 6,1 (2s, =CH₂), 5,0 (m, >CHO), 4,2 (t, CH₂O), 3,5 (t, CH₂N), 1,9 (s, CH₃) und 1,1 bis 1,3 ppm (breit, CH₂).
IR (Film): 3365 (N-H), 2835 (C-H), 1720 (C=O) und 1637 cm⁻¹ (C=C).

### Beispiel 5: Synthese eines polymerisationsfähigen Wachses durch Umsetzung des Hoechst-Wachses KST mit IEMA

Eine Lösung von 15 g (10 mmol) Hoechst-Wachs KST, 2,1 g (20 mmol) IEMA und 2 Tropfen Di-n-Octylzinndilaurat als Katalysator (Metatin 812, Firma Acima AG) in 225 ml Toluol wird 3 Tage bei 60°C gerührt. Nach Einengen der Lösung auf 80 ml im Vakuum läßt man die Mischung über Nacht im Kühlschrank stehen. Der gebildete Niederschlag wird abfiltriert, mit etwas angesäuertem Wasser und mit Ethanol gewaschen und anschließend im Exsikkator über wasserfreiem CaCl₂ getrocknet. Es ergeben sich 6,7 g (Ausbeute: 45%) eines wachsartigen Feststoffs (Schmelzpunkt: 45°C). Die Auswertung des ¹H-NMR-Spektrums zeigt, daß die OH-Gruppen praktisch quantitativ mit IEMA umgesetzt wurden.
¹H-NMR (CDCl₃): δ = 5,7 und 5,0 (2s, =CH₂), 5,0 (breit, NH), 4,3 (t, CH₂O), 3,6 (breit, CH₂), 3,5 (m, CH₂N), 2,0 ppm (s, CH₃).
IR (KBr): 2918, 2849 (C-H), 1737 (C=O) und 1637 cm⁻¹ (C=C).

### Beispiel 6: Substanzpolymerisation der wachsartigen Monomere

Je 2 g wachsartiges Monomer aus den voranstehenden Beispielen werden mit jeweils 10 mg Dibenzoylperoxid versetzt. Anschließend wird mittels DSC die sich bei der Aufheizung von Raumtemperatur auf 200°C (10°C/min) ergebende Polymerisationsenthalpie bestimmt. Auf der Grundlage der Polymerisationsenthalpie von Laurylmethacrylat als Standard ergibt sich für die Wachse aus den Beispielen 1 und 4 ein vollständiger C=C-Umsatz, während für die Wachse aus den Beispielen 2, 3 bzw. 5 ein C=C-Umsatz von 78, 69 bzw. 42 mol-% resultiert. Die C=C-Umsätze zeigen, daß sich die Methacrylatgruppen der wachsartigen Monomeren radikalisch polymerisieren lassen. Darüber hinaus wurde aus der Differenz der pyknometrisch bestimmten Dichten von Wachsmonomer und Polymer der während der Polymerisation stattfindende Volumenschrumpf (Δ-V) bestimmt. Die Δ-V-Werte liegen im Bereich von minimal - 0,7 Vol.-% (Monomer aus Beispiel 2) und maximal - 2,2 Vol.-% (Monomer aus Beispiel 4).

### Beispiel 7: Rezepturen und Eigenschaften von wachsartigen Dentalmaterialien auf der Basis von wachsartigen Monomeren

Mittel eines 3-Walzenstuhls werden die in Tabelle 1 aufgeführten Dentalmaterialien hergestellt (alle Gehaltsangaben in Gew.-%).

Aus den Dentalmaterialien werden anschließend Prüfkörper geformt und durch Bestrahlen im Spectramat (Firma Vivadent, 10 min) gehärtet. Die mit der Biegeprüfung gemäß DIN 53452 und 53457 erhaltenen Eigenschaftswerte sind in der Tabelle 2 angegeben.

**Tabelle 1**

| **Zusammensetzung von Dentalmaterialien** | | | |
|---|---|---|---|
| Komponente | Beispiel 7a | Beispiel 7b | Beispiel 7c |
| Wachsmonomer | Beispiel 1: 72,5 % | Beispiel 3: 72,0 % | Beispiel 2: 66,3 % |
| SR-295^{a)} | 26,7 % | 27,2 % | 32,9% |
| Campferchinon | 0,3 % | 0,3 % | 0,3 % |
| CEMA^{b)} | 0,5 % | 0,5 % | 0,5 % |

| | | | |
|---|---|---|---|
| ^{a)} Pentaerythrittetraacrylat | | | |
| ^{b)} 2-Cyanoethylmethylanilin | | | |

**Tabelle 2**

| **Eigenschaften von Dentalmaterialien** | | | |
|---|---|---|---|
| | Beispiel 7a | Beispiel 7b | Beispiel 7c |
| Biegefestigkeit (MPa) | 5 | 20 | 12 |
| Biege-E-Modul (MPa) | 1010 | 1320 | 1940 |

Mit dem Material gemäß Beispiel 7c wurden nach der üblichen Wachs-Methode Kronen und Inlays modelliert, die anschließend im Spectramat ausgehärtet wurden. Es wurden feste Modelle mit einer sehr guten Dimensionsstabilität und einer im Vergleich zu herkömmlichen Wachsmodellen ausgezeichneten Wärmeformbeständigkeit erhalten.

### Beispiel 8: Vergleichsbeispiel

Analog zu Beispiel 7 wurden unter Verwendung herkömmlicher, nicht polymerisierbarer Wachse Dentalmaterialien hergestellt (Tabelle 3, alle Angaben in Gew.-%).

Aufgrund der geringen Festigkeit der Materialien war die Herstellung von Prüfkörpern nicht möglich, nach dem Bestrahlen zeigten die Materialien eine starke Rißbildung. Mechanische Untersuchungen konnten daher nicht durchgeführt werden.

**Tabelle 3**

| **Zusammensetzung von Vergleichsmaterialien mit nicht-polymerisierbaren Wachsen** | | |
|---|---|---|
| Komponente | Beispiel 8a | Beispiel 8b |
| Wachsmonomer | Hoechst-Wachs S 66,1 % | S-U-Ästhetikwachs-O^{c)} 66,1 % |
| SR-295^{a)} | 33,1 % | 33,1 % |
| Campferchinon | 0,3 % | 0,3 % |
| CEMA^{b)} | 0,5 % | 0,5 % |

| | | |
|---|---|---|
| ^{a)} Pentaerythrittetraacrylat | | |
| ^{b)} 2-Cyanoethylmethylanilin | | |
| ^{c)} Firma Schuler-Dental | | |

### Beispiel 9: Vergleichsbeispiel

Gemäß US-A-5 403 188 (Beispiel 1, Run-No. 1, 12, 13, 16 und 17) wurden Dentalmaterialien mit den in Tabelle 4 gezeigten Zusammensetzungenhergestellt. Hierbei wurden keine wachsartigen Materialien erhalten, sondern thermoplastische Materialien, die beim Aufschmelzen gummiartig zäh sind. Darüberhinaus sind die Schmelzen hochviskos (Polycaprolacton TONE 767: ca. 8500 Pa·s oberhalb des Schmelzpunkts (65°C)) und nichttropfend, während die erfindungsgemäßen Wachsschmelzen eine geringe Viskosität zeigen (Beispiel 1: ca. 0,5 Pa·s oberhalb des Schmelzbereichs) und tropfbar sind.

**Tabelle 4**

| **Thermoplastische Dentalmaterialien** | | |
|---|---|---|
| TONE P-767^{a)} Gew.-% | Acrylat Gew.-% | Viskosität bei 65°C (Pa·s) |
| 100 | 0 | 8500 |
| 70 | 30^{b)} | 1140 |
| 55 | 45^{b)} | 1540 |
| 70 | 30^{c)} | 100 |
| 55 | 45^{c)} | 30 |

| | | |
|---|---|---|
| ^{a)} Polycaprolacton; Molmasse: 40 000 g/mol (Union Carbide) | | |
| ^{b)} Ebercryl 230, aliphatisches Urethandiacrylat-Harz (UCB Chemicals) | | |
| ^{c)} Ebercryl 830, hexafunktionelles Polyester-Acrylat-Oligomer (UCB Chemicals) | | |

## Patentansprüche

1. Polymerisierbares Dentalmaterial, enthaltend
(a) 0 bis 70 Gew.-% mindestens eines polymerisierbaren Monomers und/oder Oligomers;
(b) 0,1 bis 5 Gew.-% mindestens eines Polymerisationsinitiators;
(c) 0 bis 60 Gew.-% eines oder mehrerer Füllstoffe,
(d) mindestens 20 Gew.-% einer wachsartigen polymerisierbaren Substanz.

2. Dentalmaterial gemäß Anspruch 1, **dadurch gekennzeichnet**, daß der Gehalt der Komponente (a) 5 bis 70 Gew.-% beträgt.

3. Dentalmaterial gemäß Anspruch 1 oder 2, **dadurch gekennzeichnet**, daß der Gehalt der Komponente (b) 0,2 bis 2,0 Gew.-% beträgt.

4. Dentalmaterial gemäß einem der Ansprüche 1 bis 3, **dadurch gekennzeichnet**, daß der Gehalt der Komponente (c) 0 bis 50 Gew.-% beträgt.

5. Dentalmaterial gemäß einem der Ansprüche 1 bis 4, **dadurch gekennzeichnet**, daß es mindestens 40 Gew.-% einer wachsartigen polymerisierbaren Substanz enthält.

6. Dentalmaterial gemäß einem der Ansprüche 1 bis 5, **dadurch gekennzeichnet**, daß die wachsartige polymerisierbare Substanz eine längerkettige Carbonssäure, ein Derivat einer solchen Carbonsäure, eine OH-funktionalisierte Verbindung und/oder ein Derivat einer OH-funktionalisierten Verbindung ist und eine oder mehrere polymerisierbare Gruppen aufweist.

7. Dentalmaterial gemäß Anspruch 6, **dadurch gekennzeichnet**, daß die wachsartige polymerisierbare Substanz der Ester einer Carbonsäure mit einem polymerisationsfähigen Alkohol ist.

8. Dentalmaterial gemäß Anspruch 7, **dadurch gekennzeichnet**, daß die Carbonsäure einen Schmelzpunkt von über 60°C aufweist.

9. Dentalmaterial gemäß Anspruch 8, **dadurch gekennzeichnet**, daß die Carbonsäure eine Kettenlänge von 16 bis 32 Kohlenstoffatomen aufweist.

10. Dentalmaterial gemäß Anspruch 6, **dadurch gekennzeichnet**, daß die wachsartige polymerisierbare Substanz der Ester eines Alkohols mit einem polymerisationsfähigen Carbonsäurederivat ist.

11. Dentalmaterial gemäß Anspruch 10, **dadurch gekennzeichnet**, daß der Alkohol einen Schmelzpunkt von über 55°C aufweist.

12. Dentalmaterial gemäß Anspruch 11, **dadurch gekennzeichnet**, daß der Alkohol eine Kettenlänge von 18 bis 32 Kohlenstoffatomen aufweist.

13. Dentalmaterial gemäß einem der Ansprüche 6 bis 12, **dadurch gekennzeichnet**, daß die polymerisationsfähigen Gruppen Methacryl-, Acryl-, Allyl-, Vinyl-, Vinylether- und/oder Styryl-Gruppen sind.

14. Verwendung einer wachsartigen polymerisierbaren Substanz zur Herstellung eines Dentalmaterials.

15. Verwendung nach Anspruch 14, **dadurch gekennzeichnet**, daß die wachsartige polymerisierbare Substanz eine längerkettige Carbonssäure, ein Derivat einer solchen Carbonsäure, eine OH-funktionalisierte Verbindung und/oder ein Derivat einer OH-funktionalisierten Verbindung ist und eine oder mehrere polymerisierbare Gruppen aufweist.

16. Verwendung eines wachsartigen polymerisierbaren Dentalmaterials gemäß einem der Ansprüche 1 bis 13 zur Herstellung von temporären oder permanenten Dentalprothesen, Inlays oder Kronen.
